# EUROPÄISCHE PATENTSCHRIFT

(11) **EP 0 788 385 B1**
(45) Veröffentlichungstag und Bekanntmachung des Hinweises auf die Patenterteilung: **10.06.1998**
(21) Anmeldenummer: 95935840.9
(22) Anmeldetag: 27.10.1995
(51) Int. Cl.: A61M 5/28

(54) **MIT FLUIDEM INHALT ZU STERILISIERENDEN EINMALSPRITZE**
FLUID CONTAINING DISPOSABLE SYRINGE FOR STERILISATION
SERINGUE JETABLE CONTENANT UNE FLUIDE ET QUI EST A STERILISER

(30) Priorität: 28.10.1994 DE 4439738
(43) Veröffentlichungstag der Anmeldung: 13.08.1997
(73) Patentinhaber: SCHERING AKTIENGESELLSCHAFT, 13353 Berlin (DE)
(72) Erfinder: SPECK, Ulrich, D-13465 Berlin (DE)
(86) Internationale Anmeldenummer: DE9501523
(87) Internationale Veröffentlichungsnummer: WO9613289

(56) Entgegenhaltungen:
- EP-A- 0 107 874
- WO-A-93/21986
- WO-A-94/26332
- CH-A- 360 168
- DE-B- 1 239 061
- GB-A- 2 249 727

## Beschreibung

Die vorliegende Erfindung betrifft eine mit fluidem Inhalt zu sterilisierenden Einmalspritze aus Kunststoff, wobei die Einmalspritze einen Spritzenzylinder mit einem vorn angeformten Düsenteil, einen zumindest einteiligen Kolben und ein im Bereich der Zylindergriffplatte angeordnetes Kolbenanschlagelement umfaßt.

Eine Einmalspritze gemäß Oberbegriff des Anspruchs 1 ist aus CH-A-360168 bekannt.

Zur parenteralen Anwendung von flüssigen Arzneimitteln wurden bisher vorrangig Ampullen und Injektionsflaschen eingesetzt. Für die Applikation der in diesen Behältnissen abgefüllten flüssigen bzw. fließfähigen Substanzen ist zusätzlich eine Injektionsspritze mit passender Injektionskanüle erforderlich. Das bedeutet allerdings, daß das Arzneimittel vor der endgültigen Verwendung in eine solche Injektionsspritze übergeleitet werden muß. Das ist nicht nur zeitraubend, sondern beinhaltet auch eine Vielzahl von Kontaminationsmöglichkeiten.

Zur Vermeidung einer Kontaminierung der flüssigen Arzneimittel sind deshalb bereits vorgefüllte Einmalspritzen auf dem Markt verfügbar. Eine derartige Einmalspritze ist aus der EP-B 0 227 401 bekannt. Dort wird ein Verfahren zur Herstellung von gefüllten, sterilen Spritzen aus Kunststoff beschrieben. Die Einmalspritze, deren Aufbau DIN 13 098 Teil 2 entspricht, wird zur Erhaltung ihrer geometrischen Form in einem besonderen Verfahren sterilisiert. Um der Verformung der Einmalspritze aus Kunststoff im Autoklaven unter dem Einfluß des hohen Spritzeninnendrucks vorzubeugen, wird der Druck im Autoklaven durch die Zufuhr komprimierter Luft bis zum Erreichen des Spritzeninnendrucks erhöht.

Dieses Verfahren erforderte neben der Druckluftpumpe einen Dampferzeuger mit geringer Trägheit, da die in den Autoklaven eingebrachte Druckluft zwar den Autoklaveninnendruck anhebt, jedoch die für die Sterilisation notwendige Temperatur erheblich senkt. Nachteilig ist auch, daß die Wärmeübertragung durch die Anwesenheit der Luft im Luft/Dampfgemisch verringert wird, was zu einer längeren Verweilzeit im Autoklaven führt.

Ein weiterer Nachteil ergibt sich aus dem Aufbau der verwendeten Einmalspritze. Sie muß durch ihre kolbenstangenseitige Öffnung mit dem Präparat befüllt werden. Bei dieser Art der Befüllung wird unter dem einzusetzenden Kolbenstopfen im Spritzenzylinder eine Gasblase eingeschlossen, die während der Sterilisation den Spritzeninnendruck zusätzlich erhöht und damit ein Herausschieben des Kolbenstopfens und/oder eine Verformung des Spritzenkörpers forciert.

Dieser Nachteil gilt auch für eine aus der US PS 5,069,670 bekannte Subkutanspritze mit teilweise gläsernem Aufbau. Die Spritze hat einen gläsernen Spritzenzylinder, eine montierte Zylindergriffplatte mit Innenbund, ein aufgesetztes Kopfelement mit einem genormten Verbindungsteil zum Anschluß von medizinischen Kanülen, einen Kolbenstopfen und ein Verschlußteil. Sie kann jedoch nicht im befüllten Zustand sterilisiert werden. Denn ihr Glaszylinder hält dem hohen Innendruck während eines Sterilisiervorgangs im Autoklaven nicht stand.

Der vorliegenden Erfindung liegt daher das Problem zugrunde, eine mit fluidem Inhalt zu sterilisierenden Einmalspritze aus Kunststoff zu entwickeln, durch das die Einmalspritze in befülltem Zustand in einem Standartautoklaven sterilisiert werden kann ohne daß aufgrund des Spritzeninnendruckes für die Spritzenteile die Gefahr des plastischen Verformens besteht. Dabei soll die Einmalspritze derart gestaltet werden, daß ein weitgehend gasfreies Befüllen ermöglicht wird. Ferner sollen die Spritzenbauteile einfach zu reinigen und zu handhaben sein. Auch sollen die aus dem Bereich des Standes der Technik bekannten Nachteile vermieden werden.

Die Erfindung ist definiert in Anspruch 1.

Die Einmalspritze kann nahezu gasfrei befüllt werden. Die Befülleinrichtung wird am vorderen, offenen Ende des Verschlußelements angeschlossen, um das mit Druck beaufschlagte Präparat über das Düsenteil in das Innere des Spritzenkörpers zu pressen. Durch den im Düsenbereich anliegenden Kolbenstopfen kann das Präparat nicht frei in den Spritzenzylinder einströmen, sondern muß den Kolbenstopfen ggf. mit der Kolbenstange vor sich herschieben. Dadurch wird u.a. ein Schäumen des Präparats verhindert, so daß nach dem Abkuppeln der Befülleinrichtung nur wenige Gasbläschen im Spritzenzylinder vorhanden sind. Das Gas stammt größtenteils aus dem Hohlraum des Düsenteils. Es steigt beim Transport der Einmalspritze zur nächsten Bearbeitungsstation auf und sammelt sich im oberen Bereich des Düsenteils. Theoretisch mühte dann der Flüssigkeitsspiegel bei vertikal ausgerichteter Einmalspritze am unteren Rand des Düsenteils liegen. Da das Präparat jedoch unter Druck eingefüllt wurde, hat sich zum einen der Spritzenkörper geringfügig gedehnt und zum anderen wurde der Kolbenstopfen minimal gestaucht. Beide elastischen Verformungen bilden sich zurück, wodurch der Flüssigkeitsspiegel im Düsenteil nahezu bis zum oberen Rand steigt. Gegebenenfalls wird das Aufsteigen der Gasblasen in den vorderen bzw. oberen Düsenteil mit Hilfe eines Ultraschallgenerators oder dergleichen unterstützt.

Der Kolbenstopfen kann auch zum Teil in das Düsenteil hineinragen, womit das dortige Restgasvolumen verringert wird.

Die Einmalspritze wird dadurch verschlossen, daß der vordere Bereich des Düsenteils beispielsweise unter partieller Wärmezufuhr plastisch verformt wird. Dabei können auch Schweiß- und Klebeverfahren angewandt werden.

Zum Abschluß wird ggf. über das Düsenteil eine Schutzkappe gestülpt. Die Schutzkappe wird am Düsenteil oder am Spritzenzylinder durch Kraft- und/oder Formschluß befestigt. Sie schützt das relativ dünne und dünnwandige Düsenteil vor mechanischer Beschädigung.

Das Düsenteil hat in seinem hinteren Bereich die Form eines Verbindungsteils mit Außenkegel nach DIN 13 090 mit oder ohne Verriegelungsteil.

Sofern der hintere Bereich des Düsenteils nur ein kegeliges Verbindungsteil nach DIN 13 090, Teil 1 ist, wird die zuvor genannte Schutzkappe zur Befestigung auf dem Außenkegel des Verbindungsteils aufgeschoben.

Der vordere Bereich des Düsenteils ist annähernd ein rohrförmiges Verschlußelement, das über das genormte Verbindungsteil übersteht. Das sog. Verschlußelement ist mindestes 3-mal länger als das Verbindungsteil. Es dient zum einen zum Adaptieren des Spritzenkörpers an der ßefülleinrichtung und dem Sammeln des nach dem Befüllen im Spritzenkörper verbleibenden Restgases und zum anderen zum düsenseitigen Verschließen des Spritzenkörpers.

Das Düsenteil weist in dem Bereich zwischen dem Verbindungsteil und dem Verschlußelement eine Taille auf. In dieser Taille wird vor der Verabreichung des Präparats das Werkzeug zum Trennen von Verbindungsteil und Verschlußelement angesetzt. Die Taille ist derart angeordnet und gestaltet, daß nach dem Abtrennen des Verschlußelements das genormte Verbindungsteil für den Anschluß der nachzuschaltenden medizinischen Geräte entsteht.
Die Innenwandung des Verschlußelements geht trotz der Taille nahtlos und ohne Dimensionsänderung in die Innenwandung des Verbindungsteils über, so daß dort kein strömungstechnisches bzw. blasenbildendes Hindernis entsteht.

Die Taille kann u.a. bei der Verwendung von spröden Werkstoffen auch als Sollbruchstelle mit hoher Kerbwirkung ausgebildet sein. In diesem Fall kann das Verschlußelement durch Abdrehen und/oder Abbrechen vom Verbindungsteil getrennt werden.

Das Kolbenanschlagelement ist ein separates Bauteil, das mit mindestens einem Kolbenstützelement in den Spritzenzylinder hineinragt oder ihn abdeckt und die Zylindergriffplatte mit mindestens einem Umgriffselement hintergreift. Mit Hilfe des Kolbenanschlagelements wird verhindert, daß beim Befüllen und Autoklavieren der Einmalspritze der Kolben bzw. der Kolbenstopfen aus dem Spritzenzylinder herausgedrückt wird. Das Kolbenanschlagelement kann hierzu beispielsweise auf die Zylindergriffplatte aufgesetzt werden, wobei Rastelemente das Kolbenanschlagelement an der Zylindergriffplatte oder dem Spritzenzylinder fixieren.

Eine andere Alternative besteht darin, im hinteren Ende des Spritzenzylinders nach innen stehende Nocken anzuformen, an denen sich der Kolben abstützen kann. Der Kolben muß dazu im Kolbenboden oder Kolbengrundkörper den Nocken entsprechende Aussparungen haben, wobei die Aussparungen mit der Teilung am Kolbenumfang sitzen wie die Nocken an der Zylinderinnenwandung. Der Kolben wird nach dem Einsetzen in den Spritzenzylinder um eine halbe Teilung um seine Längsachse geschwenkt, so daß Kolbenbodenbereiche ohne Aussparunq mit den Nocken zur Deckung kommen.

Das Kolbenanschlagelement ist zumindest im Bereich des Spritzenzylinders mit Aussparungen versehen, damit keine geschlossenen Hohlräume zwischen dem Kolben und dem Kolbenanschlagelement liegen. Derartige Hohlräume könnten beim Autoklavieren aufgrund ihrer thermischen Isolierwirkung partiell eine für die Sterilisation ungenügende Erwärmung bewirken.

Das gleiche Problem betrifft auch die Schutzkappe mit dem darunterliegenden Düsenteil. Damit das Düsenteil genügend erwärmt wird, hat die Schutzkappe außerhalb ihres Befestigungsbereiches Ausnehmungen.
Auch ermöglichen die Ausnehmungen und Ausbrüche ein vollständiges Trocknen der Einmalspritze nach dem Autoklavieren.

Das Befüllen der Einmalspritze über das Düsenteil benötigt aufgrund des querschnittsbedingt geringen Volumenstroms mehr Zeit als das rückseitige Befüllen des Spritzenzylinders. Damit die Taktzeit des herkömmlichen Befüllverfahrens erhalten bleibt, werden vorzugsweise mehrere Befülleinrichtungen parallel beschickt.

Weitere Einzelheiten der Erfindung ergeben sich aus dem nachfolgend beschriebenen und schematisch dargestellten Ausführungsbeispiel.
- Figur 1:: Teilgeschnittene Seitenansicht der montierten Einmalspritze vor dem Befüllen;
- Figur 2:: Draufsicht auf die teilgeschnittene, montierte Einmalspritze;
- Figur 3:: Teilgeschnittene Seitenansicht des Düsenteils mit Luer-Lock-Verbindungsteil vor dem Befüllen der Einmalspritze;
- Figur 4:: Teilgeschnittene Seitenansicht des Düsenteils nach dem Befüllen und Verschließen der Einmalspritze.

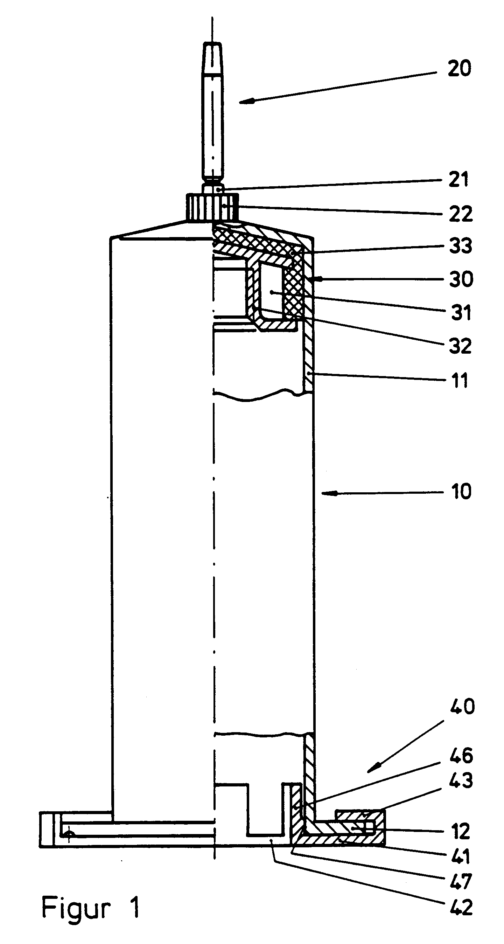

Figur 1 zeigt eine großvolumige Einmalspritze, die abgesehen von einem besonderen Düsenteil (20) und einem Kolbenanschlagelement (40) der DIN 13 098. Teil 1 für Einmalspritzen aus Kunststoff entspricht. Demnach besteht ihr Spritzenkörper (10) aus einem Spritzenzylinder (11) mit einer am hinteren Ende angeformten Zylindergriffplatte (12) und einem am vorderen Ende angeordneten verriegelbaren Verbindungsteil (21) mit Außenkegel nach DIN 13 090, Teil 2. Allerdings ist das Verbindungsteil (21) im Rahmen der Erfindung u.a. verlängert ausgeführt. Im Spritzenzylinder (11) sitzt ein Kolbenstopfen (30). Dieser Kolbenstopfen (30) besteht aus mit einem Innengewinde (32) und einer über den Kolbengrundkörper (31) gestülpten Kolbendichtung (33). Das Innengewinde (32) dient zur Befestigung der nicht dargestellten Kolbenstange. Der Kolbengrundkörper (31) ragt an seinem hinteren Ende in der Spritzenlängsrichtung über die Kolbendichtung (33) über, um eine definierte Auflage für das Aufliegen auf dem Kolbenanschlagelement (40) zu bilden.

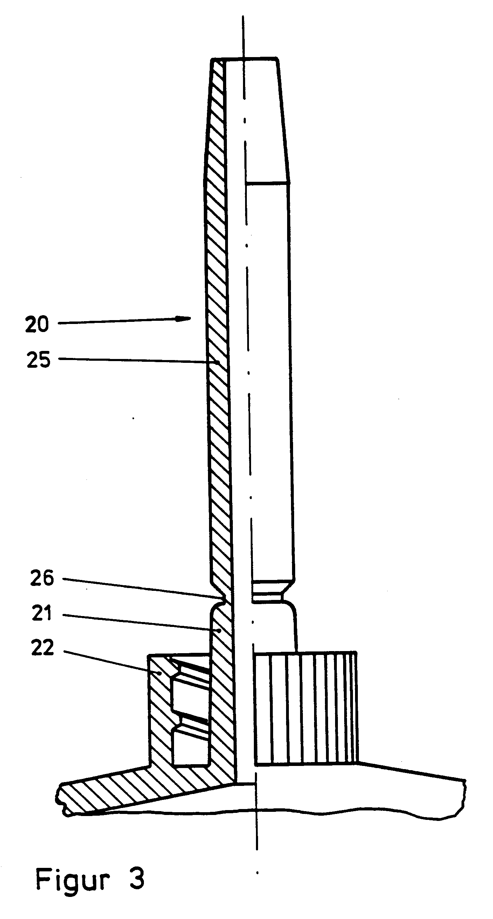

In Figur 3 ist das Düsenteil (20) der unbefüllten Einmalspritze vergrößert dargestellt. Es besteht neben dem genormten Verriegelungsteil (22) und dem kegeligen Verbindungsteil (21) mit einer u.a. für die Befüllung vorgesehenen Verlängerung. Die Verlängerung, die ca. 3-mal so lang ist wie das Verbindungsteil (21), ist ein sog. Verschlußelement (25). Das Verschlußelement (25) und das Verbindungsteil (21) trennt eine Taille (26). Zum Verbindungsteil (21) hin ist die Flanke der Taille (26) ein an die Form des Verbindungsteils (21) angepaßter Außenradius. Der Taillengrund ist zylindrisch. Dort liegt die Wandstärke unter einem halben Millimeter. Zum Verschlußelement (25) hin ist die Taillenflanke eine 45°-Fase.

Das Verschlußelement (25) hat an seinem vorderen Ende einen Außenkegel mit einer Neigung 1:4. Die Innenwandung des gesamten Düsenteils (20) ist annähernd zylindrisch.

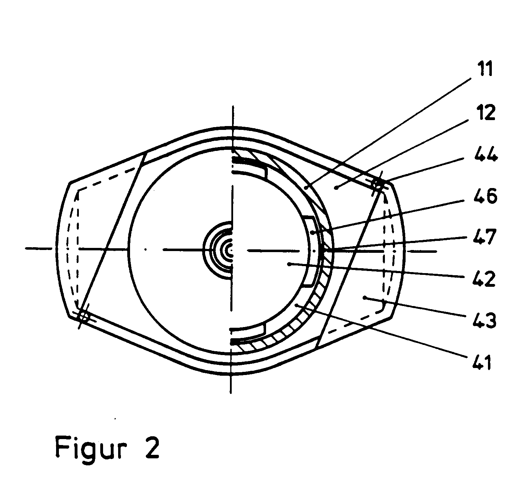

Am anderen Ende der Einmalspritze sitzt im Spritzenzylinder (11) das Kolbenanschlagelement (40), vgl. Figur 1 und 2. Es umfaßt eine Grundplatte (41), auf der zum einen vier in den Spritzenzylinder (11) hineinragende Kolbenstützelemente (46) angeordnet sind und zum anderen zwei Umgriffelemente (43), die zumindest teilweise die Zylindergriffplatte (12) außen umgreifen. Die Kolbenstützelemente (46) sind in einem äquidistanten Abstand um eine zentrale Bohrung (42) der Grundplatte (41) herum angeformt. Jedes der Kolbenstützelemente (46) hat über seine gesamte Länge einen Querschnitt, der weitgehend der Form eines Kreisringabschnitts entspricht. Die Außenkontur des Kreisringquerschnittes hat ca. die Länge von 1/8 des Kreisumfangs. Die nicht gekrümmten Seitenflächen der Kolbenstützelemente (46) bilden Ebenen, die um die halbe Breite der Kolbenstützelemente (46) parallel versetzt zur gedachten Mittellinie der Einmalspritze verlaufen. Die parallel zur Wandung des Spritzenzylinders (11) ausgerichteten Kolbenstützelemente (46) berühren die Wandung selbst nicht. Das hat u.a. den Vorteil daß nach dem Sterilisieren diese Zonen schnell abtrocknen.

Zwischen den Kolbenstützelementen (46) und der Wandung des Spritzenzylinders (11) befinden sich Zentrierelemente (47). Letztere sitzen an der Außenkontur der Kolbenstützelemente (46) im Bereich der Zylindergriffplatte (12). Sie sind parallel zur Spritzenmittellinie und mittig zu dem jeweiligen Kolbenstützelement (46) ausgerichtet. Ihr Querschnitt ist annähernd halbkreisförmig.

Die Grundplatte (41), deren zentrale Bohrung (42) auch der Führung der nicht dargestellten Kolbenstange dient, deckt von außen her die Zylindergriffplatte (12) vollständig ab. Dabei steht sie über den Rand der Zylindergriffplatte (12) geringfügig über. Der überstehende Rand ist in dem von der Spritzenmittellinie am entferntesten liegenden Bereich abschnittsweise kreisförmig ausgebildet. U.a. in diesem Randbereich sind die Umgriffelemente (43) angeformt. Die Anformungen erstrecken sich nach Figur 2 bei beiden Hälften der Zylindergriffplatte (12) zusätzlich in die Richtung des Spritzenzylinders (11). Entsprechend der Figur 2 ist dies an der rechten Hälfte im unteren und in der linken Hälfte im oberen Bereich. Die Umgriffelemente (43) umgreifen von außen her teilweise die Unterseite der Zylindergriffplatte (12), so daß die Unterseite auf beiden Hälften jeweils schräg abgedeckt wird. Die Form des Kolbenanschlagelements ergibt sich zum Teil aus der Montageart. Das Kolbenanschlagelement (40) wird, vergleichbar mit einem Bajonettverschluß, zunächst in den Spritzenzylinder eingesteckt und dann bezüglich der Einsteckrichtung durch eine Rechtsdrehung um 90° in die Umgriffsposition gebracht. Dazu ist das Kolbenanschlagelement (40) bei der Montage gegenüber der Darstellungen in Figur 1 und 2 bezüglich der Spritzenmittellinie um 90° gedreht in den Spritzenzylinder (11) eingesetzt. Erst durch die Rechtsdrehung gelangt das Kolbenanschlagelement (40) in die dargestellte Position. Zur Fixierung in dieser Umgriffsposition sind am Rand der beiden Umgriffelemente (43) jeweils zwei Sicherungsnoppen (44) angeordnet. Sie verhindern ein unbeabsichtigtes Zurückdrehen des Kolbenanschlagelements (40).

Vor dem Befüllen der Einmalspritze werden nach dem Sterilisieren der Einzelteile und dem Aufbringen des Gleitmittels auf die Innenwandung des Spritzenzylinders der Kolbenstopfen (30) in den Spritzenkörper (10) eingesetzt und bis an das vordere Ende des Spritzenzylinders geschoben. Dann wird das Kolbenanschlagelement (40) eingesteckt und zur Arretierung geschwenkt. Beim Einsetzen des Kolbenstopfen (30) und/oder des Kolbenanschlagelements (40) kann je nach Bauart auch die originäre Kolbenstange montiert sein. Auch kann beim Einsetzen des Kolbenstopfens (30) das Kolbenanschlagelement (40) schon lose auf der Kolbenstange sitzen.

Zum Befüllen der Einmalspritze wird beispielsweise die mit einem Innenkegel ausgestattete Befülldüse der Abfüllmaschine auf den Außenkegel des Verschlußelements (25) aufgesetzt. Während des Befüllvorganges wird der Kolbenstopfen (30) von dem in den Spritzenzylinder (11) hineingepreßten flüssigen Präparat in Richtung Kolbenanschlagelement (40) geschoben. Sobald der Kolbenstopfen (30) auf dem Kolbenanschlagelement (40) aufliegt. ist der Befüllvorgang beendet und die Befülldüse hebt vom Verschlußelement (25) ab. Auf dem Transport der Einmalspritze zur nächsten Bearbeitungsposition steigen durch die Transporterschütterungen oder durch zusätzlich aufgebrachte Vibrationen im Präparat eingeschlossene Gasblasen auf und sammeln sich im vorderen Bereich des Verschlußelements (25). Der vordere Teil wird unter Zufuhr von Hitze zugequetscht, abgedreht oder in vergleichbarer Art unter plastischer Verformung dicht verschlossen.

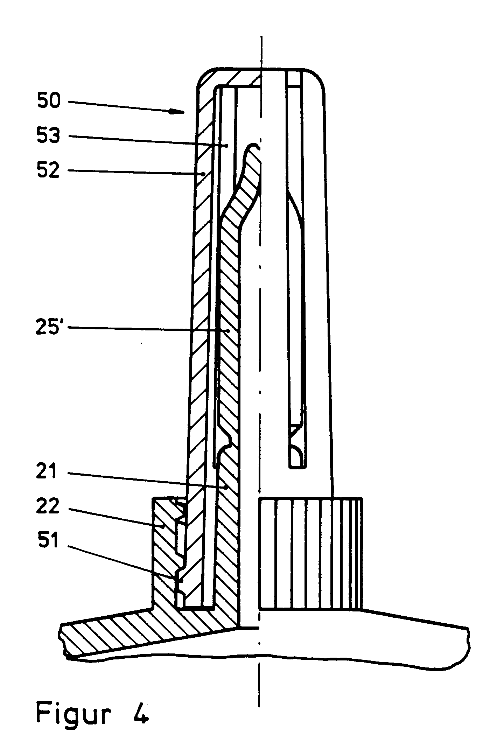

Das verformte Verschlußelement (25') ist in Figur 4 dargestellt. Über das Verschlußelement (25') wird eine kegelstumpfförmige Schutzkappe (50) gestülpt und mit Hilfe des im Verriegelungsteil (22) integrierten Gewindes festgeschraubt. Am unteren Rand der Schutzkappe (50) sitzen dazu zwei kurze Gewindeabschnitte (51), wie sie beispielsweise DIN 13 090, Teil 2 entnehmbar sind, vgl. Verbindungsteil LLS mit Form A oder B. Die Schutzkappe (50) hat vier zur Spritzenmittellinie symmetrisch angeordnete Ausnehmungen (53), die sich über 3/4 der Schutzkappenlänge erstrecken.

Zum Vorbereiten der Anwendung der Einmalspritze wird die Schutzkappe (50) abgeschraubt und das Verschlußelement (25') vom kegeligen Verbindungsteil (21) abgetrennt. Das Abtrennen im Bereich der Taille (26) kann mit Hilfe einer Schere, eines Messers, eines Skalpells oder dgl. erfolgen. Nach dem Abtrennen des Verschlußelements (25') entspricht das Düsenteil (20) bezüglich seiner geometrischen Form der DIN 13 090.

### BEZUGSZEICHENLISTE

- 10: Spritzenkörper
- 11: Spritzenzylinder
- 12: Zylindergriffplatte
- 20: Düsenteil
- 21: kegeliges Verbindungsteil
- 22: Verriegelungsteil
- 25: Verschlußelement, unverformt
- 25': Verschlußelement, verformt
- 26: Taille
- 30: Kolbenstopfen, Kolben
- 31: Kolbengrundkörper
- 32: Gewinde für Kolbenstange
- 33: Kolbendichtung
- 40: Kolbenanschlagelement
- 41: Grundplatte
- 42: Bohrung, zentral
- 43: Umgriffelemente
- 44: Sicherungsnoppen
- 46: Kolbenstützelemente
- 47: Zentrierelemente
- 50: Schutzkappe
- 51: Gewindeabschnitt
- 52: Stege
- 53: Ausnehmungen

## Patentansprüche

1. Mit fluidem Inhalt zu sterilisierende Einmalspritze aus Kunststoff, die einen Spritzenzylinder (11) mit vorn angeformtem Düsenteil (20), einen zumindest einteiligen Kolben oder Kolbenstopfen (30) und ein im Bereich der Zylindergriffplatte (12) angeordnetes Kolbenanschlagelement (40) umfaßt, dadurch gekennzeichnet,
- daß das Düsenteil (20) in seinem hinteren Bereich die Form eines Verbindungsteils (21) mit Außenkegel nach DIN 13 090 Teil 2 mit oder ohne Verriegelungsteil (22) hat,
- daß das Düsenteil (20) in seinem vorderen Bereich ein annähernd rohrförmiges Verschlußelement (25, 25') ist, das im Bereich seines freien, äußeren indes unter partieller Wärmezufuhr plastisch verformbar ist und
- daß das Düsenteil (20) in dem Bereich zwischen dem Verbindungsteil (21) und dem Verschlußelement (25, 25') eine Taille (26) aufweist.

2. Einmalspritze gemäß Anspruch 1, dadurch gekennzeichnet, daß über das Düsenteil (20) eine Schutzkappe (50) stülpbar ist, wobei die Schutzkappe (50) am Düsenteil (20) oder am Spritzenzylinder (11) durch Kraft- und/oder Formschluß befestigbar ist.

3. Einmalspritze gemäß Anspruch 1, dadurch gekennzeichnet, daß das Kolbenanschlagelement (40) ein separates Bauteil ist, das mit mindestens einem Kolbenstützelement (46) in den Spritzenzylinder (11) hineinragt oder ihn abdeckt und die Zylindergriffplatte (12) mit mindestens einem Umgriffelement (43) hintergreift.

4. Einmalspritze gemäß Anspruch 2, dadurch gekennzeichnet, daß die Schutzkappe (50) außerhalb ihres Befestigungsbereiches Ausnehmungen (53) aufweist.

## Claims

1. Plastics, fluid-containing disposable syringe that is to be sterilised, comprising a syringe cylinder (11) having a nozzle portion (20) formed at the front, a plunger or plunger stopper (30) that is in at least one piece and a plunger stop element (40) that is arranged in the region of the cylinder grip plate (12), characterised in that
- the nozzle portion (20), in its rear region, is in the form of a connecting portion (21) having a male taper according to DIN 13 090, Part 2, with or without a locking portion (22),
- the nozzle portion (20), in its front region, is an approximately tubular sealing element (25, 25') that can be deformed plastically in the region of its free, outer end with local supply of heat,
- the nozzle portion (20) has a waist (26) in the region between the connecting portion (21) and the sealing element (25, 25').

2. Disposable syringe according to claim 1, characterised in that a protective cap (50) can be fitted over the nozzle portion (20), it being possible for the protective cap (50) to be fastened to the nozzle portion (20) or to the syringe cylinder (11) by non-positive and/or positive fit.

3. Disposable syringe according to claim 1, characterised in that the plunger stop element (40) is a separate component that projects by at least one plunger support element (46) into the syringe cylinder (11) or covers it and that grips underneath the cylinder grip plate (12) by at least one grip-round element (43).

4. Disposable syringe according to claim 2, characterised in that the protective cap (50) has recesses (53) outside its fastening region.

## Revendications

1. Seringue en matière plastique jetable, à stériliser avec le contenu liquide, comprenant un cylindre d'injection (11) avec une partie frontale servant d'ajoutage (20) au moins un piston ou bouchon-piston tout d'une pièce (30) et un moyen servant à actionner le piston (40) disposé dans la région de la platine de préhension du cylindre (12), caractérisée en ce que
- l'ajoutage (20) présente dans sa partie arrière la forme d'un élément de raccord (21) avec un cône extérieur selon la norme DIN 13 090, partie 2, avec ou sans moyen de verrouillage (22),
- l'ajoutage (20) présente dans sa partie frontale un moyen de fermeture (25, 25') approximativement cylindrique, lequel moyen, dans la région de son extrémité externe, libre, peut subir une déformation élastique sous l'effet d'un apport partiel de chaleur, et
- l'ajoutage (20) présente un col (26) dans la région située entre l'élement de raccord (21) et le moyen de fermeture (25, 25').

2. Seringue jetable selon la revendication 1, caractérisée en ce qu'un capot de protection (50) peut être enfoncé sur l'ajoutage (20), le capot de protection (50) pouvant être fixé l'ajoutage (20) ou sur le cylindre de seringue (11) par pression ou mécaniquement.

3. Seringue jetable selon la revendication 1, caractérisée en ce que le moyen servant à actionner le piston (40) est un composant indépendant, lequel pénètre dans ou couvre le cylindre de syringue (11) à l'aide d'au moins un moyen de support de piston (46), et saisit la platine de préhension du cylindre (12) par derrière à l'aide d'au moins un élément enveloppant (43).

4. Seringue jetable selon la revendication 2, caractérisée en ce que le capot protecteur (50) présente des évidements (53) en dehors de sa région de fixation.
